# EUROPEAN PATENT APPLICATION

(11) **EP 2 692 849 A1**
(43) Date of publication of application: **05.02.2014**
(21) Application number: 12762975.6
(22) Date of filing: 06.02.2012
(51) Int. Cl.: C12M 1/14

(54) **CELL-ADHESIVE PHOTOCONTROLLABLE BASE MATERIAL**

(30) Priority: 30.03.2011 JP 2011076462
(71) Applicant: The University of Tokyo, Bunkyo-Ku Tokyo 113-8654 (JP); Hitachi High-Technologies Corporation, Tokyo 105-8717 (JP)
(72) Inventor: KONNO Tomohiro, Tokyo 113-8654 (JP); ISHIHARA Kazuhiko, Tokyo 113-8654 (JP); BYAMBAA Batzaya, Tokyo 113-8654 (JP); SUGIYAMA Hisashi, Hitachinaka-shi Ibaraki 312-8504 (JP); OZAWA Satoshi, Kokubunji-shi Tokyo 185-8601 (JP)
(74) Representative: Denison, Christopher Marcus
(86) International application number: PCT/JP2012/052597
(87) International publication number: WO 2012/132551

(57) **Abstract**

In analyzing, fractionating, and culturing cells alive, operations can be more simply made in real time and culture can be performed while removing unnecessary cells from cultured cells for purification. Desired cells are also analyzed and fractionated from the cultured cells to increase the purity, recovery rate, and viability of the cells. A cell-adhesive photocontrollable base material is used in which light irradiation causes the bond dissociation of a photolabile group comprising an O-nitrobenzyl skeleton to irreversibly change the surface of the irradiated portion thereof from that of the cell-adhesive material to that of a non-cell-adhesive material. Cell images are detected and analyzed to obtain the positional information of desired cells. Based on this information, areas among cells and a cell-adhesive photocontrollable material are cut by second light irradiation. On the other hand, the surface of the base material is changed from a cell-adhesive one to a non-cell-adhesive one by first light irradiation to produce the detachment between cells and the base material. This enables cells to be analyzed and fractionated alive.

## Description

### Technical Field

The present invention relates to the fields of regenerative medicine and stem-cell research, and particularly relates to a technique for analysis, fractionation and culture of cells.

### Background Art

In the field of regenerative medicine, operations are performed which involve identifying and isolating only a few somatic stem cells or progenitor cells contained in somatic cells and culturing the resultant to prepare somatic cells. Culture is also attempted for obtaining somatic stem cells or somatic cells by differentiation induction from iPS cells or ES cells as a source for somatic stem cells. However, the iPS cells or ES cells are not uniform and cells differentiation-induced therefrom are also not uniform. Various cells occur. Cells or tissues used for regenerative medicine are required to have uniformity as somatic cells, to contain somatic stem cells, and to be free of cancer cells or cancer stem cells or pluripotent stem cells such as iPS cells and ES cells.

Thus, techniques for analyzing, fractionating, and culturing cells become increasingly important in the field of regenerative medicine. To fractionate cell groups of a plurality of types, an analysis technique is necessary for discriminating them. In addition, the molecular biological properties or cell biological properties of a single type of cells cannot be analyzed unless the single type of cells is fractionated from the discriminated cell groups of a plurality of types. Differentiation induction can be strictly controlled only when these requirements are satisfied. Further, a technique is required for removing unnecessary cells when a differentiation induction efficiency of 100% is not achieved.

Devices for analyzing cells alive include a well-known light microscope, a fluorescence microscope for observing fluorescently labeled cells, and a fluorometric imaging device; however, these devices cannot fractionate cells. On the other hand, devices for fractionating cells alive include an device for fractionating and collecting desired cells by the antigen-antibody reaction between an antigen on the cell surface and an antibody added to magnetic beads; however, this device cannot analyze cells and has a problem in the purity, recovery rate, and the like thereof. Devices for fractionating cells also include a laser microdissection device; however, it is mainly used for isolation from a dead cell section embedded in paraffin.

Devices for analyzing and fractionating cells alive include a flow cytometer and a sorting device which are well known. These devices are each an device which analyzes and discriminate cells by exposing the individual cells in a sample stream imposed on a sheath stream to laser light and observing scattered light or fluorescence, followed by giving charges to droplets containing the individual cells based on the information and performing fractionation and applying the electric field. A multicolored laser light can be irradiated to analyze many fluorescent markers; however, this is cumbersome in fluorescence correction and optical axis adjustment. When trypsin treatment or the like is performed to separate cell masses into individual cells in advance, the cells are not a little damaged. In addition, although the sorted cells have high purity and a high recovery rate, there are problems including that the viability thereof is reduced by impact during sorting. For treatment using these devices, cells must be once removed from a culture base material.

Techniques for analyzing, fractionating, and culturing cells alive include a method as described in Patent Literature 1. This technique is associated with a device for using a cell culture base material on which a photoresponsive material whose physical properties are changed by light irradiation is film-formed, discriminating between cultured cells with a monitor, locating desired cells, subjecting the desired cell position to light pattern irradiation, and detaching the desired cells from the culture base material. As the "photoresponsive material whose physical properties are changed by light irradiation" described there, one is mentioned which has a function by which cells are detached from the culture base material by the isomerization of the structure thereof by light irradiation to change the polarizability and hydophilic-hydrophbic property thereof; particularly, changes in these physical properties are considered to be preferably reversible.

### Citation List

### Patent Literature

Patent Literature 1: JP Patent No. 3975266
Patent Literature 2: JP Patent No. 3472723
Patent Literature 3: JP Patent Publication (Kokai) No. 2004-170930 A
Patent Literature 4: JP Patent Publication (Kokai) No. 2008-167695 A

### Non Patent Literature

Non Patent Literature 1: Kazuhiko Ishihara, Seitai Zairyo (Biocompatible Material) 18 (1): 33 (2000).
Non Patent Literature 2: Y. Arima et al., J. Meter. Chem., 17, 4079(2007).
Non Patent Literature 3: Y. Arima et al., Biomaterials 28, 3074(2007).
Non Patent Literature 4: M. N. Yousaf et al., PNAS 98(11), 5992(2001).
Non Patent Literature 5: Toshiaki Furuta, Kogaku (Optics) 34 (4): 213 (2005)
Non Patent Literature 6: J. Edahiro et al., Biomacromolecules, 6(2), 970(2005).
Non Patent Literature 7: J. Nakanishi et al., Analytical Sciences, 24, 67 (2008).

### Summary of Invention

### Technical Problem

As the photoresponsive material whose physical properties are changed by light irradiation, described in the above JP No. 3975266, the material whose structure is reversibly changed by light is difficult to be caused to 100% have one of the two isomers, which reduces the selectivity of cell adhesion. The material responsive to light of a long wavelength as described in the examples therein will be changed in adhesion, for example, by responding to exciting light for fluorescent observation. In addition, while the technique can detach cells from a culture base material, it does not contemplate detachment of the adhesion between cells. Thus, the technique will wholly detach isolated cells or a cell mass present in the culture base material, leaving a problem that a cell mass consisting of a plurality of types of cells adhering to each other cannot be fractionated to single cells.

In view of the foregoing prior art, the present invention is directed to provide a cell-adhesive photocontrollable base material for analyzing, fractionating, and culturing cells alive.

An object of the present invention is to enable simpler operation in real time and culture while removing unnecessary cells from cultured cells for purification in analyzing, fractionating, and culturing the cells alive and to analyze and fractionate desired cells from the cultured cells to increase the purity, recovery rate, and viability of the cells as compared to before.

### Solution to Problem

To solve the above prior art problems, the present invention has adopted the following means.

That is, the cell-adhesive photocontrollable base material of the present invention is obtained by film-forming a cell-adhesive photocontrollable material in which a cell-adhesive material is bonded to a non-cell-adhesive material via a photolabile group, on a base material.

In the cell-adhesive photocontrollable base material of the present invention, light irradiation causes the bond dissociation of a photolabile group to produce the separation of a cell-adhesive material to leave a non-cell-adhesive material.

In the cell-adhesive photocontrollable base material of the present invention, light irradiation also causes the bond dissociation of a photolabile group to irreversibly change the surface of the irradiated portion thereof from that of the cell-adhesive material to that of a non-cell-adhesive material.

A method for analyzing and fractionating cells using the cell-adhesive photocontrollable base material of the present invention comprises the following steps.

A step of seeding and culturing cells on a cell-adhesive photocontrollable base material obtained by film-forming a cell-adhesive photocontrollable material in which a cell-adhesive material is bonded to a non-cell-adhesive material via a photolabile group, on a base material, or a cell-adhesive photocontrollable base material, wherein light irradiation causes the bond dissociation of a photolabile group to produce the separation of a cell-adhesive material to leave a non-cell-adhesive material, or a cell-adhesive photocontrollable base material, wherein light irradiation causes the bond dissociation of a photolabile group to irreversibly change the surface of the irradiated portion thereof from that of the cell-adhesive material to that of a non-cell-adhesive material.

A step of detaching and recovering desired cells from the base material by first light irradiation to desired cellular regions.

A method for analyzing and fractionating cells using the cell-adhesive photocontrollable base material of the present invention also comprises the following step.

A step of providing cell-adhesive regions and a non-cell-adhesive region by first light irradiation on a cell-adhesive photocontrollable base material obtained by film-forming a cell-adhesive photocontrollable material in which a cell-adhesive material is bonded to a non-cell-adhesive material via a photolabile group, on a base material, or a cell-adhesive photocontrollable base material, wherein light irradiation causes the bond dissociation of a photolabile group to separate a cell-adhesive material to leave a non-cell-adhesive material, or a cell-adhesive photocontrollable base material, wherein light irradiation causes the bond dissociation of a photolabile group to irreversibly change the surface of the irradiated portion thereof from that of the cell-adhesive material to that of a non-cell-adhesive material.

A device for analyzing and fractionating cells using the cell-adhesive photocontrollable base material of the present invention comprises a cell-adhesive photocontrollable base material obtained by film-forming a cell-adhesive photocontrollable material in which a cell-adhesive material is bonded to a non-cell-adhesive material via a photolabile group, on a base material, or a cell-adhesive photocontrollable base material, wherein light irradiation causes the bond dissociation of a photolabile group to separate a cell-adhesive material to leave a non-cell-adhesive material, or a cell-adhesive photocontrollable base material, wherein light irradiation causes the bond dissociation of a photolabile group to irreversibly change the surface of the irradiated portion thereof from that of the cell-adhesive material to that of a non-cell-adhesive material, and
a first light irradiation means for subjecting the cell-adhesive photocontrollable material on the base material to photoreaction.

The present specification encompasses the contents of the specification and/or drawings of Japanese Patent Application No. 2011-076462 on which the priority of the present application is based.

### Advantageous Effects of Invention

In analyzing, fractionating, and culturing cells alive, operations can be more simply made in real time and culture can be performed while removing unnecessary cells from cultured cells for purification. Desired cells can also be analyzed and fractionated from the cultured cells to increase the purity, recovery rate, and viability of the cells.

### Brief Description of Drawings

[Figure 1] Figure 1 is a diagram showing a method (1) for analyzing cells during culture and fractionating desired cells.
[Figure 2] Figure 2 is a diagram showing a method (2) for analyzing individually separated cells and fractionating desired cells.
[Figure 3] Figure 3 is a diagram showing a method (3) for analyzing individually separated cells and fractionating desired cells.
[Figure 4] Figure 4 is a chart showing a ¹H-NMR spectrum of the methacrylic acid ester monomer represented by formula (14).
[Figure 5] Figure 5 is a chart showing an IR spectrum of the methacrylic acid ester monomer represented by formula (14).
[Figure 6] Figure 6 is a chart showing a ¹H-NMR spectrum of the methacrylic acid ester tercopolymer represented by formula (15).
[Figure 7] Figure 7 is a chart showing an IR spectrum of the methacrylic acid ester tercopolymer represented by formula (15).
[Figure 8] Figure 8 is a graph showing the adhesion of HELA cells and their desorption due to light irradiation by the number of cells.
[Figure 9] Figure 9 is a graph showing comparison of proliferative capacity between normal and light irradiation-desorbed HELA cells.

### Description of Embodiments

Embodiments of the present invention will be described below. However, the present invention is not intended to be limited thereto.

One embodiment of the present invention is a cell-adhesive photocontrollable base material obtained by film-forming a cell-adhesive photocontrollable material in which a cell-adhesive material is bonded to a non-cell-adhesive material via a photolabile group, on a base material. In the cell-adhesive photocontrollable material, light irradiation can cause the bond dissociation of the photolabile group to produce the separation of the cell-adhesive material from the base material. The light bond dissociation may occur between the non-cell-adhesive material and the photolabile group or between the photolabile group and the cell-adhesive material. The light irradiation leaves the non-cell-adhesive material in the base material. The irreversible photodissociation reaction can efficiently change the cell-adhesive one into the non-cell-adhesive one, enabling the enhancement of adhesion selectivity.

The cell-adhesive photocontrollable base material described above can be used to analyze and fractionate particular cells. As used herein, the analysis and fractionation refers to analyzing cells and fractionating them from other cells.

Examples of the non-cell-adhesive material include a biocompatible material with a phosphorylcholine group, having a structure similar to that of a cell membrane. The non-cell-adhesive material is, for example, a (meth)acrylic ester polymer with a phosphorylcholine group, represented by general formula (1) below.

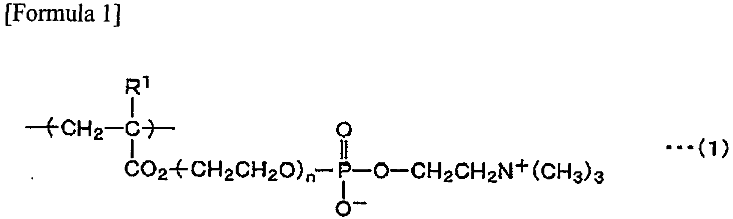

In the formula, R¹ represents hydrogen or a methyl group and n represents a number of 1 to 20.

A (meth)acrylic ester polymer represented by general formula (2) below may also be used as the non-cell-adhesive material.

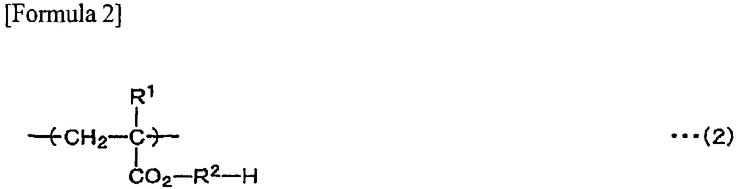

In the formula, R¹ is the same as that in the general formula (1), and R² represents 1 to 20 alkylene groups or 1 to 20 polyoxyethylene groups.

The non-cell-adhesive material may be a copolymer of (meth)acrylic ester polymers represented by the general formulas (1) and (2). In addition, an alkoxysilane represented by general formula (3) below can be used as the non-cell-adhesive material.
[Formula 3]

(R³O)₃Si-R²-H (3)

In the formula, R² is the same as that in the general formula (2), and R³ represents hydrogen or an alkyl group.

Examples of the cell-adhesive material include a material having a cell-adhesive group in the terminal end. Examples of the cell-adhesive group include a group represented by general formula (4) below.
[Formula 4]

-X (4)

In the formula, X represents a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, or a nucleic acid group.

The cell-adhesive group X of the general formula (4) can be varied to provide a variation in adhesion to various cells. In addition, the cell-adhesive material encompasses a material in which an extracellular matrix promoting adhesion to cells or an antibody capable of bonding to a surface antigen of cells and a protein or the like for the bonding of the antibody thereto bonds or adheres to a group represented by the general formula (4) described above. Examples of the extracellular matrix include collagens, non-collagenous glycoproteins (fibronectin, vitronectin, laminin, nidogen, teneinosine, thrombospondi, von Willebrand, osteopontin, fibrinogen, and the like), elastins, and proteoglycans. Examples of the protein capable of causing the antibody to bond include avidin/biotin, protein A, and protein G. The antibody can also be caused to bond using an avidin/biotin system.

The photolabile group can be dissociated by reaction to light of particular wavelength. The wavelength of the photoreaction of the photolabile group should be 360 nm or more which is non-cytotoxic and a shorter wavelength than the wavelength of incident light for light microscopical observation or exciting light for fluorescent observation. This can ensure that a change in adhesion due to light for cell observation does not occur during cell observation. Examples of the photolabile group include an O-nitrobenzyl group, a hydroxyphenacyl group, and a coumarinylmethyl group; however, one can be preferably used which comprises an O-nitrobenzyl skeleton represented by divalent general formula (5) below.

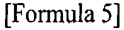

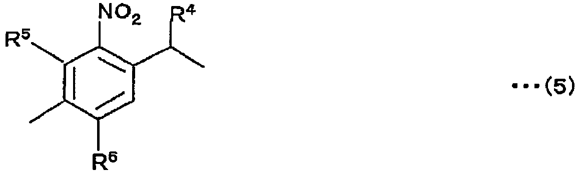

Here, in the formula, R⁴ may be hydrogen, a halogen group, a hydroxy group, an alkyl group, a carboxylic acid group, a carboxylic acid ester group, an amide group, an amino group, or the like, and R⁵ and R⁶ may each independently be hydrogen, an alkoxy group, a vinyloxy group, or the like. The mutual positional relationship between meta and para positions may be changed. In addition, the benzene ring may also be changed into a naphthalene ring.

The photolabile group and the cell-adhesive material form a structure in which a cell-adhesive group represented by the general formula (4) directly or indirectly bonds to a photolabile group represented by the general formula (5) at a position on the benzene skeleton or at the benzyl position. Photodissociation occurs at the benzyl position. For example, when bonding is performed at a position on the benezene skeleton, the bonding may be carried out via a divalent linking group R⁷, as represented by general formula (6) below.

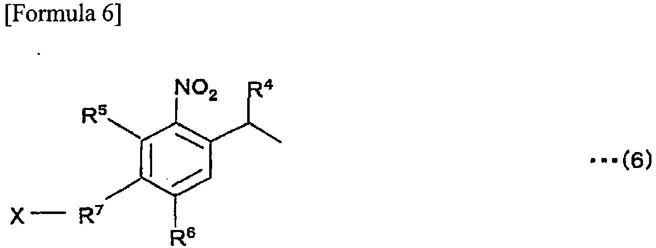

Here, R⁴, R⁵, and R⁶ are the same as those in the general formula (5).

As the divalent linking group R⁷, O(CH₂)m, O(CH₂CH₂O)ₘ, OCO(CH₂)ₘ, OCOCH₂O(CH₂CH₂O)ₘ (where m is an integer of 0 to 20), or the like may be used; however, R⁷ has only the action of causing the photolabile group to bond to the cell-adhesive group. If the direction of the photolabile group is reversed, photodissociation can occur between the photolabile group and the cell-adhesive group. Examples thereof can include a structure represented by general formula (7) below as a structure in which the bonding is made to a divalent linking group R⁸ at the benzyl position.

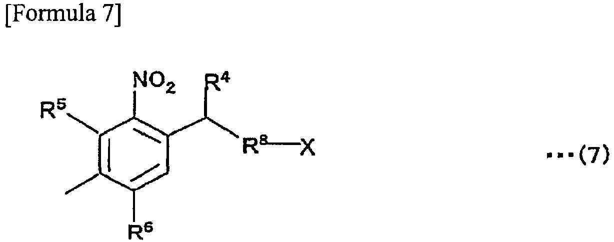

Here, R⁴, R⁵, and R⁶ are the same as those in the general formula (5).

As the divalent linking group R⁸, O, OCO, NH, OCONH, NHCO, S, or the like may be used; however, R⁸ has only the action of causing the photolabile group to bond to the cell-adhesive group.

The structure in which the cell-adhesive material bonds to the photolabile group as described above is directly or indirectly bonded to the non-cell-adhesive material.

For example, the structure may be made in the form of a (meth)acrylic ester polymer represented by general formula (8) or (9) below and incorporated into the non-cell-adhesive material.

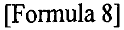

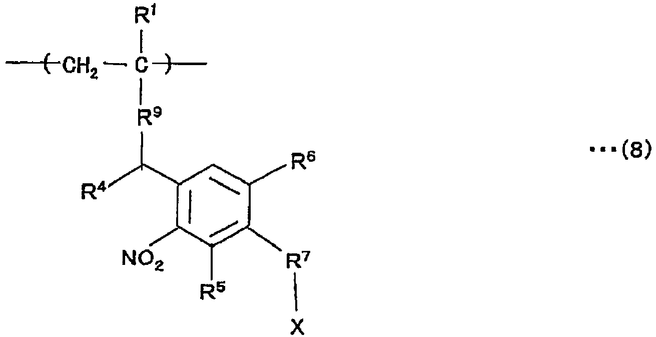

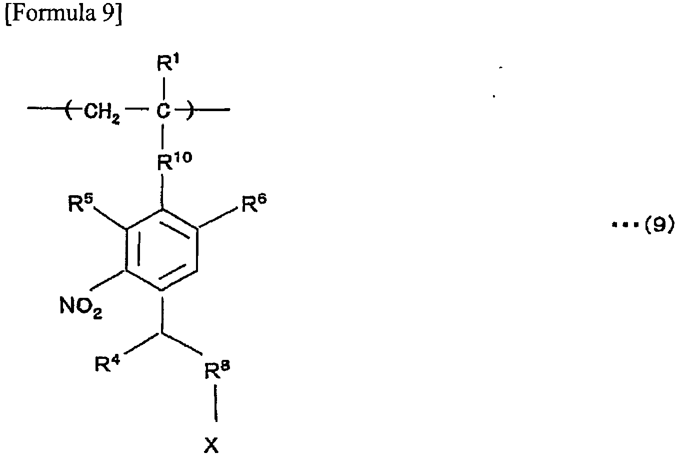

Here, R¹, R⁴, R⁵, R⁶, R⁷, and R⁸ are as described above. R⁹ and R¹⁰ represent divalent linking groups, and may be O, CO₂, CONH, CO₂(CH₂CH₂O)ₚ, CO₂(CH₂CH₂)ₚO, CONH(CH₂CH₂O)ₚ, CONH(CH₂CH₂)ₚO (p is an integer of 1 to 20), or the like; however, R⁹ and R¹⁰ each have only the action of linking a structure having the cell-adhesive material bonded to the photolabile group to the non-cell-adhesive material.

Examples of the material in which the structure having the cell-adhesive group bonded to the photolabile group according to the present invention is bonded to the non-cell-adhesive material can include a copolymer of (meth)acrylic ester represented by the general formulas (1) and (8) described above; the general formulas (1) and (9) described above; the general formulas (1), (2), and (8) described above; or the general formulas (1), (2), and (9) described above. The copolymer can be made to optionally change the ratio of the cell-adhesive material to the non-cell-adhesive material, and the changed ratio results in providing a variation in adhesion to various cells. The copolymerization of a (meth)acrylic ester containing an alkoxysilane at the side chain of each of these polymers can increase adhesion to the base material.

Although these systems take the form of copolymers, they may be used in the form of homopolymers. For example, a system having a structure represented by the general formula (8) or (9) described above may be used alone. A material in which each of these structures having cell-adhesive groups bonded to photolabile groups is bonded to a non-cell-adhesive material can be film-formed on a base material to produce a cell-adhesive photocontrollable base material.

The system can also be made in the form of an alkoxysilane represented by general formula (10) or (11) below and film-formed on a base material by silane coupling, followed by producing a cell-adhesive photocontrollable base material bonded to a compound having a structure into which a cell-adhesive group X represented by general formula (12) or (13) below is introduced. Its production by such a step can prevent the hydrolysis of the alkoxysilane due to the preintroduction of the cell-adhesive group X. Although maleimide groups bond to R⁷ and R⁸ in the compounds represented by the general formulas (10) and (11), the portion of each maleimide group may be converted into -X represented by the general formula (4) described above.

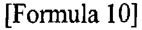

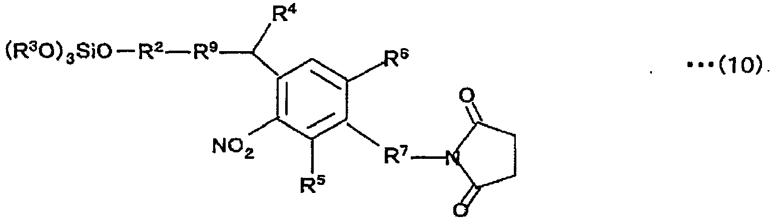

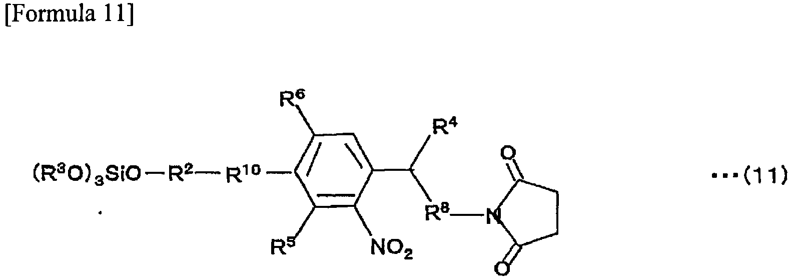

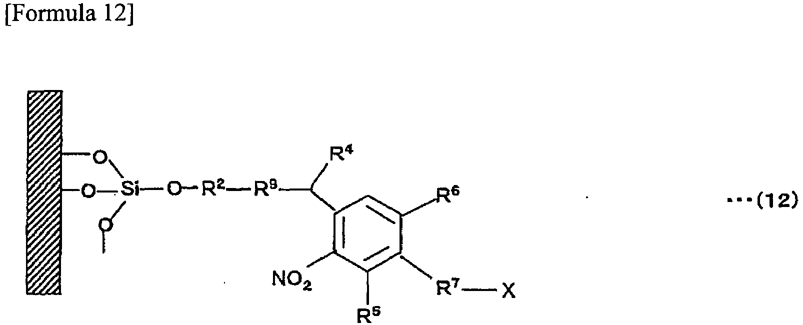

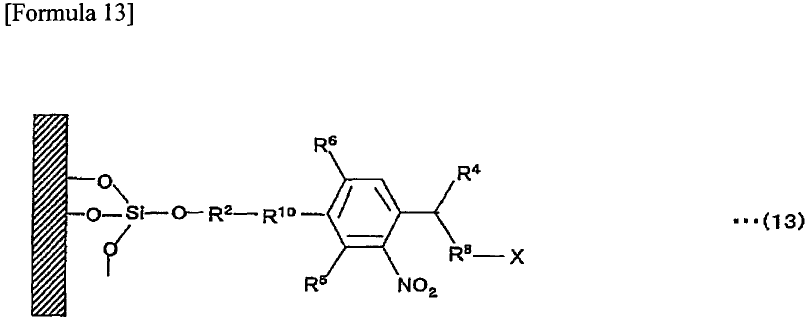

These cell-adhesive materials again include a material in which an extracellular matrix promoting adhesion to cells or an antibody capable of bonding to a surface antigen of cells and a protein or the like for bonding to the antibody bonds or adheres thereto.

The base material for film-forming each of the cell-adhesive photocontrollable materials thereon may be a transparent plastic culture vessel or the like; however, a transparent glass culture vessel may be preferably used in view of optical performance and durability.

The method for analyzing and fractionating cells using the cell-adhesive photocontrollable base material will now be described in detail based on figures.

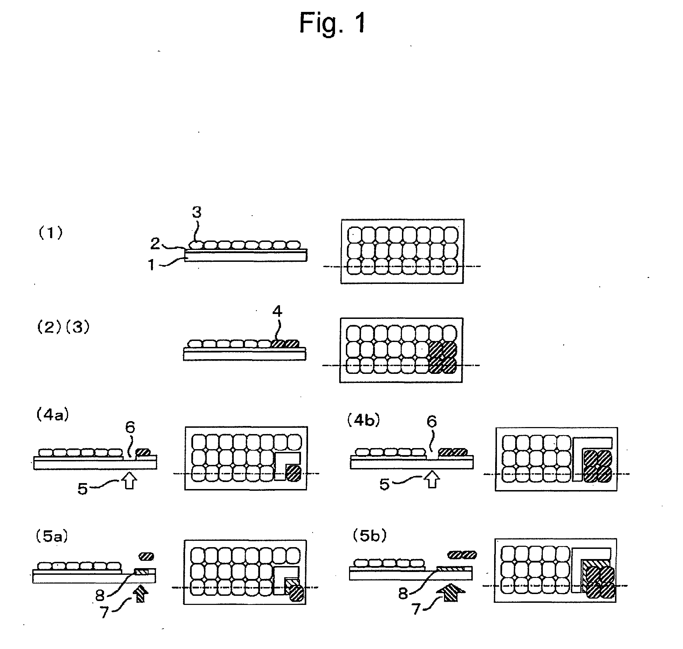

Figure 1 illustrates one aspect of the method for analyzing and fractionating cells using the cell-adhesive photocontrollable base material of the present invention. The method consists of steps (1), (2), (3), (4a), and (5a) or consists of steps (1), (2), (3), (4b), and (5b). In Figure 1, each step is illustrated in the set of right and left figures, and the left figure is a cross section of the portion indicated by a dashed line in the right figure. The description of the steps is as follows. (1) Cells 3 are seeded and cultured on a cell-adhesive photocontrollable material 2 film-formed on a glass culture vessel (transparent base material) 1. (2) Cell images are detected by microscopic observation (including the observation of transmission images, phase contrast images, differential interference images, and the like), fluorescent observation, scattered light observation, Raman light observation, or the like; a characteristic amount of cells are extracted; and then (3) desired cells are identified and the positional information of the cells is obtained. The desired cells include necessary cells to be analyzed and fractionated and unnecessary cells. Here, cells are labeled with a fluorescent marker or the like; however, the fluorescent marker labeling or the like may be performed before or after culture. (4a) In the example of this aspect, the cells 4 shown in (3) are unnecessary cells, and the other cells 3 are necessary cells. The periphery of the side of the cell 4 and a cell-adhesive photocontrollable material 6 in the boundary between the cells 3 and the cells 4 are subjected to second light irradiation 5 for cutting. As a result, the cells 4 in the portion indicated by a rectangle in (4a) are destroyed. Laser light can be preferably used for the second light irradiation 5 here. (5a) When the area on the base material in which the cells 4 are present is wide, first light irradiation 7 is performed on the region 8 in which the cells 4 are present to change the cell-adhesive photocontrollable material 2 into a non-cell-adhesive one, and the remaining cells 4 are detached from the glass culture vessel 1 and recovered together with the culture solution. When the area on the base material in which the cells 4 are present is narrow, the second light irradiation 5 may be performed on all cells 4 and a cell-adhesive photocontrollable material 8 for cutting/destruction. Thereafter, the culture is continued, and the cells 3 left on the base material may continue to be cultured while sequentially removing unnecessary cells 4 for purification.

On the other hand, a step (4b) in Figure 1 is a step when the cells 4 are desired to be fractionated/isolated for analysis (when the cells 4 are necessary). The periphery of the side of the cells 3 in the boundary between the cell 3 and the cell 4 and the cell-adhesive photocontrollable material 6 are subjected to the second light irradiation 5 for cutting.

Laser light can be preferably used for the second light irradiation 5. Thereafter, (5b) the region 8 on the base material in which the cells 4 are present is subjected to the first light irradiation 7 to change the cell-adhesive photocontrollable material 2 into a non-cell-adhesive one, and the cells 4 are detached from the glass culture vessel 1 and recovered together with the culture solution. The recovered fractionated cells can be analyzed alive.

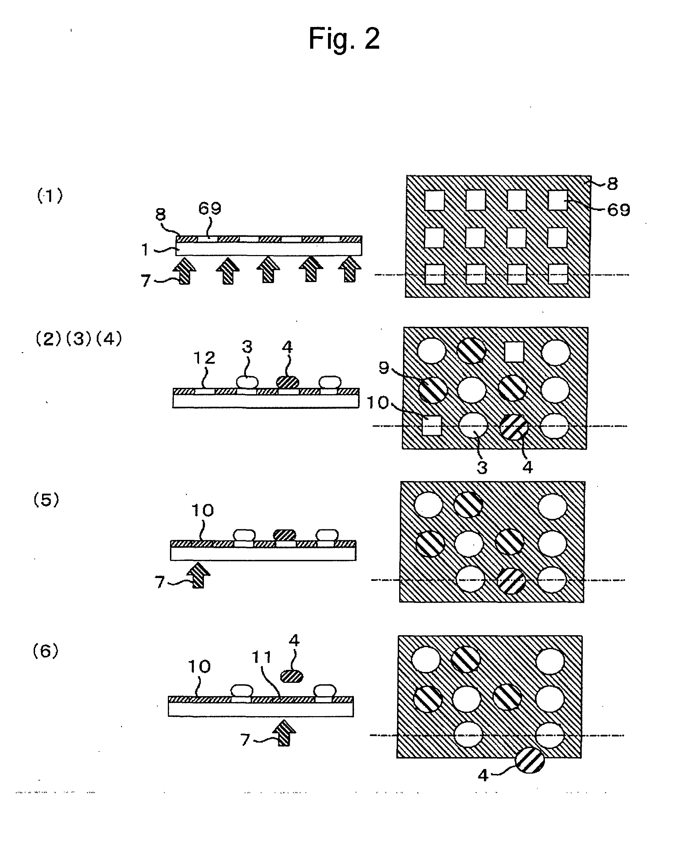

Figure 2 illustrates another aspect of the method for analyzing and fractionating cells using the cell-adhesive photocontrollable base material of the present invention. The method consists of steps (1), (2), (3), (4), (5), and (6). In Figure 2, each step is illustrated in the set of right and left figures, and the left figure is a cross section of the portion indicated by a dashed line in the right figure. (1) The cell-adhesive photocontrollable material film-formed on a glass culture vessel 1 is subjected to first light irradiation 7 as shown in the figure to provide cell-adhesive regions 69 and a non-cell-adhesive region 8. The cell-adhesive regions 69 and the non-cell-adhesive region 8 can be set together to any pattern by changing the irradiation pattern of light. In the example shown in Figure 2, the cell-adhesive regions 69 were arranged in a lattice form as areas of single cells. In other words, the first light irradiation 7 was performed so that the cell-adhesive regions 69 were arranged in a lattice form. Here, addresses are preferably allocated so that the cell-adhesive regions can be identified.

(2) An already cultured cell mass is then separated into individual cells by treatment with trypsin or the like and seeded on the base material. (3) Cell images are detected by microscopic observation (including the observation of transmission images, phase contrast images, differential interference images, and the like), fluorescent observation, scattered light observation, Raman light observation, or the like; a characteristic amount of cells are extracted; and then (4) desired cells are identified and the positional information of the cells is obtained. The desired cells include necessary cells to be analyzed and fractionated and unnecessary cells. Here, cells are labeled with a fluorescent marker or the like; however, the fluorescent marker labeling or the like may be performed before or after seeding. In the example shown in Figure 2, it is assumed that in addition to the cells 3, other cells (for example, cells 4 and cells 9) are present. (5) When cells do not adhere to all addresses (the cell-adhesive regions 69), the address regions to which no cells adhere are subjected to the first light irradiation 7 to make them non-cell-adhesive (the region of reference symbol 10 shown in the figure). (6) Then, a region 11 of a desired cell, a cell 4 here, is subjected to the first light irradiation 7, and the cell 4 is detached from the glass culture vessel 1 and recovered together with the culture solution. The step (6) can be sequentially repeated to further fractionate and isolate the cells 3 and 9.

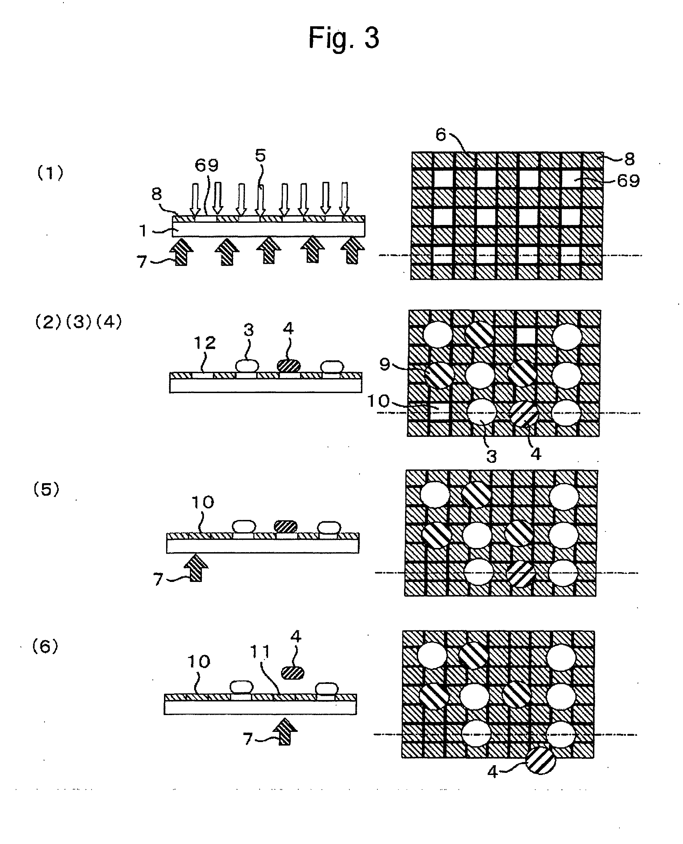

Figure 3 illustrates still another aspect of the method for analyzing and fractionating cells using the cell-adhesive photocontrollable base material of the present invention. The method consists of steps (1), (2), (3), (4), (5), and (6). In Figure 3, each step is illustrated in the set of right and left figures, and the left figure is a cross section of the portion indicated by a dashed line in the right figure. (1) A cell-adhesive photocontrollable material 2 film-formed on a glass culture vessel 1 is subjected to second light irradiation (for example, irradiation with laser light) 5 in columns and rows at predetermined intervals to cut the adjacent cell-adhesive photocontrollable material (the cutting-plane line is indicated by symbol 6 in Figure 3). Predetermined positions are also subjected to first light irradiation 7 to provide cell-adhesive regions 69 and non-cell-adhesive regions 8. Here, addresses are preferably allocated so that the cell-adhesive regions can be identified.

The cell-adhesive regions 69 and the non-cell-adhesive regions 8 can be set together to any pattern by changing the irradiation pattern of light; however, here, the cell-adhesive regions 69 were arranged in a lattice form as areas of single cells. (2) An already cultured cell mass is then separated into individual cells by treatment with trypsin or the like and seeded on the base material. (3) Cell images are detected by microscopic observation (including the observation of transmission images, phase contrast images, differential interference images, and the like), fluorescent observation, scattered light observation, Raman light observation, or the like; a characteristic amount of cells are extracted; and then (4) desired cells are identified and the positional information of the cells is obtained. The desired cells include necessary cells to be analyzed and fractionated and unnecessary cells. Here, cells are labeled with a fluorescent marker or the like; however, the fluorescent marker labeling or the like may be performed before or after seeding. In the example shown in Figure 3, it is assumed that in addition to cells 3, cells 4 and cells 9 are present.

(5) When cells do not adhere to all cell-adhesive regions (addresses) 69, the address 10 region to which no cell adheres is subjected to the first light irradiation 7 to make it non-cell-adhesive. (6) Then, a region 11 in which a desired cell, a cell 4 here, is present is subjected to the first light irradiation 7, and the cell 4 is detached from the glass culture vessel 1 and recovered together with the culture solution. The step (6) can be sequentially repeated to further fractionate and isolate the cells 3 and 9. The difference with Figure 2 lies in that whereas in the method shown in Figure 2, the first light irradiation 7 by itself does not cut the cell-adhesive material into the regions when a fibrous or membranous material such as an extracellular matrix or feeder cells is present on the upper layer of the cell-adhesive material, in the method shown in Figure 3, the second light irradiation 5 is performed to cut it between the regions.

The device for performing the method for analyzing and fractionating cells using the cell-adhesive photocontrollable base material (for example, a cell-adhesive photocontrollable material film-formed on a transparent base material) of the present invention at least comprises (1), (2), (3), (4), (6), and (7) below:

(1) the cell-adhesive photocontrollable base material;
(2) a stage on which the base material is placed;
(3) an optical detection means for obtaining cell images;
(4) a means for obtaining positional information from cell images;
(5) a second light irradiation means for cutting or destructing areas among cells and a cell-adhesive photocontrollable material;
(6) a first light irradiation means for subjecting the cell-adhesive photocontrollable material on the base material to photoreaction to make it non-cell-adhesive; and
(7) a means for controlling the motion of each means.

The optical detection means for obtaining cell images described in (3) above may use a well-known optical system. In obtaining cell images, light is irradiated which has a wavelength not affecting the photolabile group of the cell-adhesive photocontrollable material. For example, using a lamp or LED providing broad emission spectra as a light source, light is irradiated through a short-wavelength cut filter for at least removing light of a wavelength not more than the photoreaction wavelength to detect transmitted light, reflected light, or the like using a 2-dimensional sensor such as CCD. In the case of fluorescent images from cells, light of the absorption wavelength range of a desired fluorochrome, having the range of a wavelength longer than the photoreaction wavelength is irradiated as an exciting light by dispersion with a bandpass interference filter or the like. Laser light of the above wavelength range may also be used. Fluorescence is detected with a 2-dimensional sensor such as CCD through a wavelength filter such as an exciting light cut filter or a fluorescence wavelength transmission filter (a bandpass interference filter or the like). If the exciting light is sharply restricted and a 2-dimensional-scanning mode is adopted, the fluorescent image can also be measured using a photomultiplier tube. Measurement can be made by switching a plurality of wavelength filters to provide fluorescent images of a plurality of fluorescence wavelengths, enabling application to a plurality of fluorophores. Passage through a dispersive element such as a prism or a diffracting grating and detection with a line sensor or the like also enable a finer wavelength spectrum image to be obtained.

The second irradiation means of (5) above can be performed by using an infrared laser or an ultraviolet laser as the light source for laser scanning based on the positional information obtained by the means of (4) above. The laser scanning uses an XY deflector and light irradiation is performed on desired positions. Light pattern irradiation can also be performed by one operation through a photomask reflecting the positional information obtained by the means of (4) above. In that case, an optical system for condensing laser light on the base material through a spatial light modulation device to avoid preparing a fixed photomask each time the experiment is performed is preferable as a pattern generator. The spatial light modulation device may be a reflex or transmissive spatial light modulation device. The reflex spatial light modulation device may be a digital mirror device, and the transmissive spatial light modulation device may be a liquid crystal spatial light modulation device. Here, the laser wavelength usable in the digital mirror device or the liquid crystal spatial light modulation device is mainly in the range from visible light to near-infrared light. Thus, a near infrared laser, which can be strongly absorbed by water, can be used as a laser light source. When the wavelength is set within the ultraviolet region, visible to near infrared laser light may be passed through a spatial light modulation device and then passed through a wavelength conversion device such as a nonlinear crystal or a ferroelectric crystal to use a second harmonic or a third harmonic, which has a 1/2 or 1/3 wavelength, respectively.

The first light irradiation means of (6) above uses a wavelength of the photoreaction of the cell-adhesive photocontrollable material as a light source. A lamp, LED, or the like providing a broad emission spectrum is used, and light of a wavelength of 360 nm or less or even light of a wavelength of not less than the fluorescence excitation wavelength is cut by a wavelength filter; or a laser of a photoreaction wavelength can be used. It is also possible that light scanning is performed using an XY deflector based on the positional information obtained by the means of (4) above for light irradiation on desired positions, or light pattern irradiation is performed by one operation through a photomask reflecting the positional information obtained by the means of (4) above. In that case, an optical system for condensing light on the base material through a spatial light modulation device to avoid preparing a fixed photomask each time the experiment is performed is preferable as a pattern generator. The spatial light modulation device may be a reflex or transmissive spatial light modulation device. The reflex spatial light modulation device may be a digital mirror device, and the transmissive spatial light modulation device may be a liquid crystal spatial light modulation device.

The optical systems of (3), (5), and (6) above preferably use parts as common as possible.

Examples for proof of principle as one aspect of the present invention will be presented below. However, the present invention is not intended to be limited thereto.

### Examples

### [Example 1]

Synthesis of Methacrylic Acid Ester Monomer Represented by Formula (14)

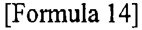

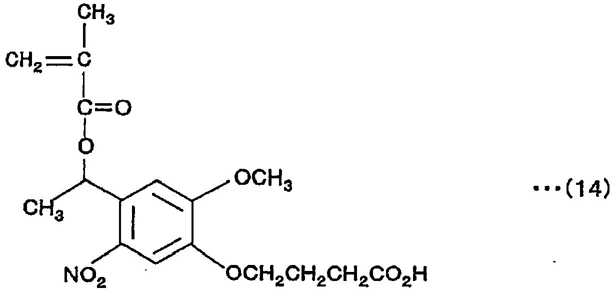

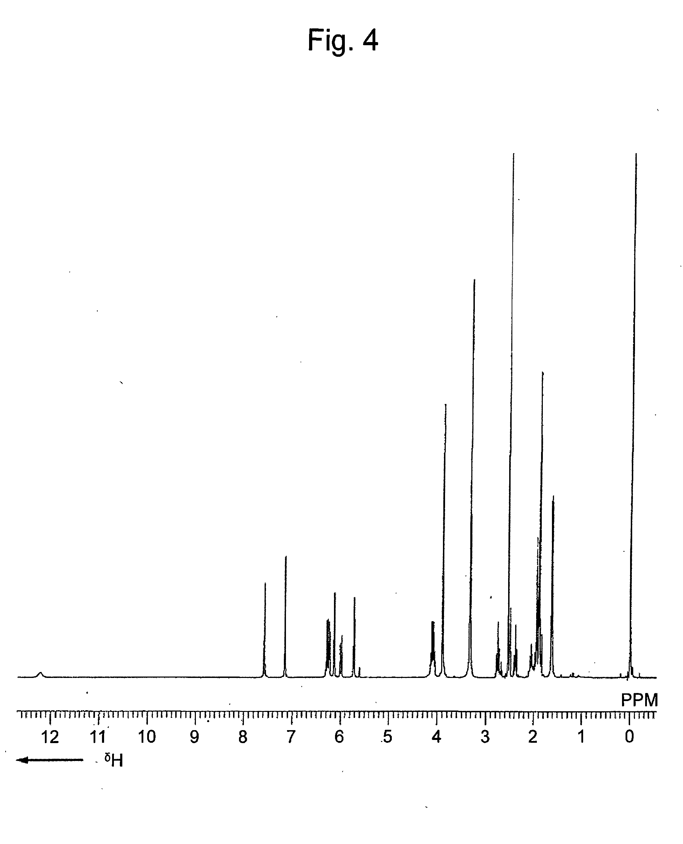

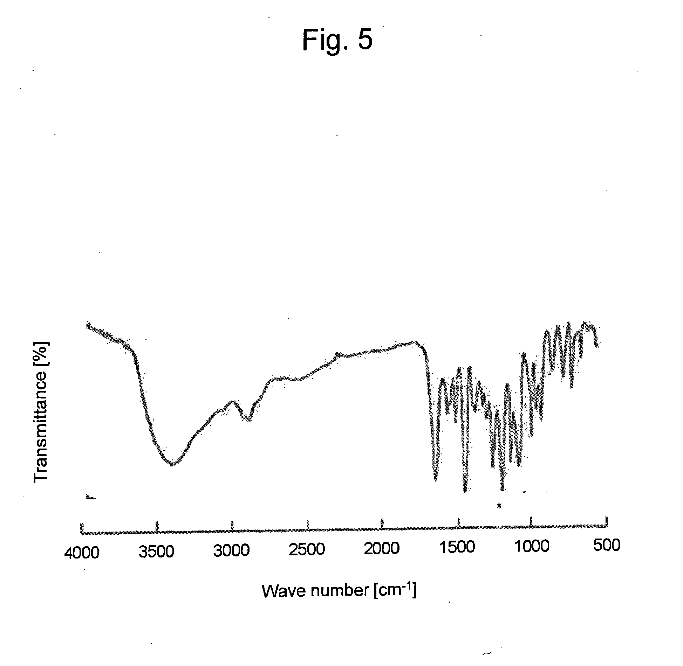

0.3 g of hydroxyethyl photo linker was dissolved in 2.56 g of methylene chloride and 0.3 g of triethylamine. The mixture was degassed with argon gas for 10 minutes. Subsequently, 0.52 g of methacrylic acid chloride was added thereto under conditions of 0°C and stirred at room temperature a whole day and night. The reaction solution was washed with a 5% sodium carbonate aqueous solution, dilute hydrochloric acid, and distilled water in that order. The volatile component was evaporated by depressurization. The resultant liquid was re-dissolved in a 50% acetone solution. The mixed solution was stirred at room temperature a whole day and night and a photolabile monomer was extracted using methylene chloride. The extracted methylene chloride layer was washed with dilute hydrochloric acid and distilled water in that order. The resultant compound was vacuum dried and then lyophilized. All of the above operations were performed in a light-shielded state. Its ¹H-NMR spectrum and IR spectrum are shown in Figures 4 and 5, respectively.

Synthesis of Methacrylic Acid Ester Tercopolymer Represented by Formula (15)

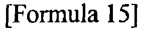

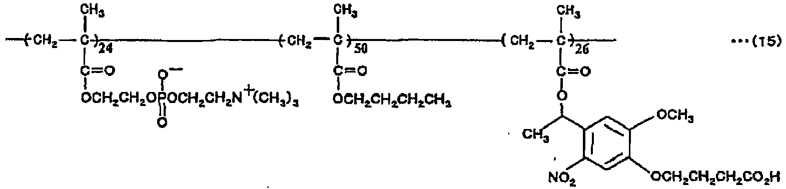

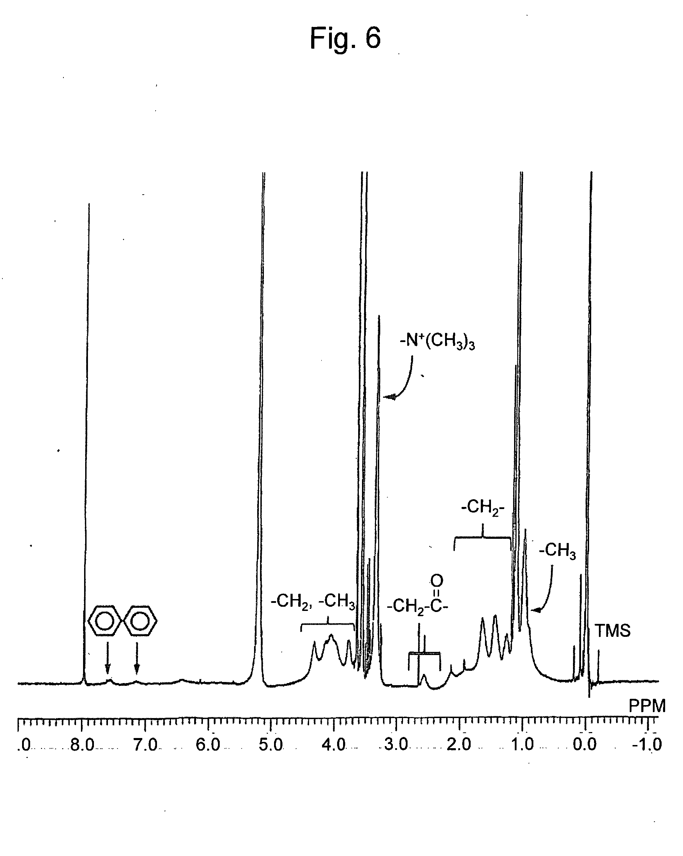

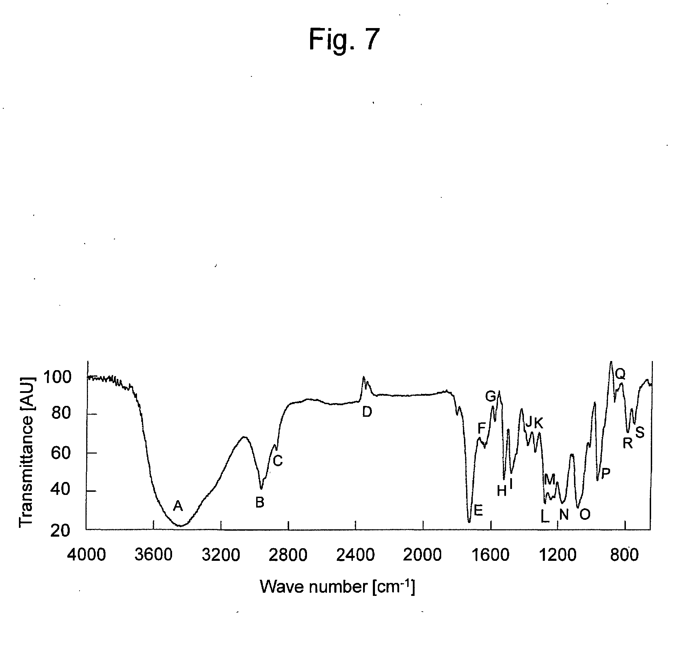

Methacrylic acid ester tercopolymer was synthesized by radical polymerization using 2,2'-azobisisobutyronitrile (polymerization initiator). A mixture of the respective monomers (25:50:25 mole%) and AIBN was charged into a polymerization tube and dissolved to a monomer concentration of 0.38 M in a mixed solvent of dioxane/ethylalcohol = 1/1 (v/v). The tube was degassed for 10 minutes using argon gas and then sealed. The polymerization tube was charged into an oil bath set to 60°C, followed by reaction for 48 hours. After reaction, the resultant was cooled to room temperature and charged into a poor solvent of diethyl ether/chloroform = 6/4 (v/v) to provide a precipitate. Mw: 1.1 x 10⁴, Mw/Mn: 1.65. Its 1H-NMR spectrum and IR spectrum are shown in Figures 6 and 7, respectively.

### Desorption of Cell by Light Irradiation on Surface of Tercopolymer Represented by Formula (15)

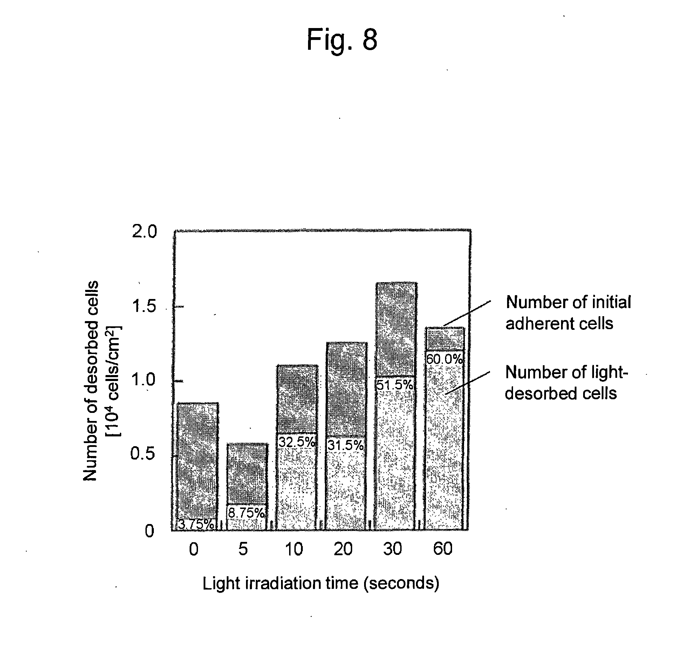

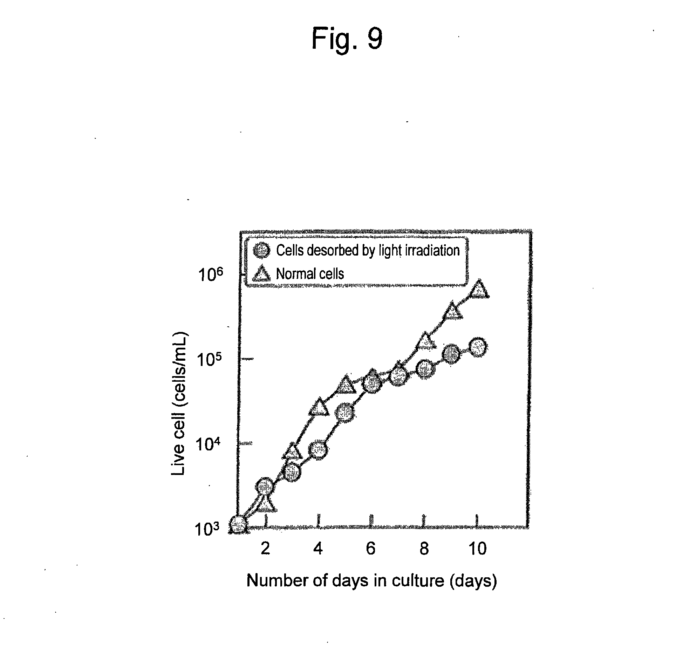

A cell-adhesive photocontrollable base material was prepared by dip coating a 0.5 wt% ethanol solution of the tercopolymer represented by the formula (15) on a glass base material. 2 x 10⁴ HeLa cells were seeded thereon and allowed to stand under conditions of 37°C and 5% CO₂ for 3 hours. Next, the resultant was irradiated with light of 365 nm (80 mW/cm²), and immediately thereafter, the supernate was recovered, followed by counting the number of cells. Then, the light-irradiated base material was washed, followed by recovering the remaining adherent cells using trypsin to count the number of cells. As a result, as shown in Figure 8, the percentage of detached cells increased with the time of light irradiation. In another experiment, after the adhesion of 91 % cells, 60-second light irradiation was found to cause the detachment of 67% cells. The number of live cells of the recovered cells decreased with increasing light irradiation time to 180 seconds, 300 seconds, and 600 seconds; however, 60 seconds had no phototoxic effect, and the recovered cells proliferated as did normal cells as shown in Figure 9.

### [Comparative Example 1]

2 x 10⁴ HeLa cells were seeded on a polystyrene culture base material and allowed to stand under conditions of 37°C and 5% CO₂ for 3 hours. Next, the resultant was irradiated with light of 365 nm (80 mW/cm²), and immediately thereafter, the supernate was recovered, followed by counting the number of cells. Then, the light-irradiated base material was washed, followed by recovering the remaining adherent cells using trypsin to count the number of cells. As a result, 86% cells adhered thereto, and 60-second light irradiation caused the detachment of 5% cells.

Although this Example is only illustrative, various types of cells can be caused to adhere by properly selecting the cell-adhesive group of the cell-adhesive photocontrollable material or properly controlling the ratio of the cell-adhesive material to the non-cell-adhesive material. The cell-adhesive photocontrollable base material is excellent in selectivity in the adhesion between cells and the base material because it is efficiently irreversibly changed from a cell-adhesive one to a non-cell-adhesive one by photodissociation reaction, and further, in recovering desired cells present in arbitrary regions, the purity and recovery rate of the cells can be increased because the adhesion among cells and the cell-adhesive photocontrollable material can be cut with a laser light. In addition, it is unnecessary that in culturing cells, the cells be once taken out of the culture base material and purified as for a flow cytometer or a sorting device, and the culture can be performed on the same culture base material while removing unnecessary cells in real time, simplifying the culture/purification operation. Even when normal cells contact or mix with abnormal cells, the abnormal cells are spatially separated from the normal cells and an abnormal cell region is compartmentalized. This enables the photodissociation reaction to be conducted by being limited to the abnormal cell region, enabling the selective detachment of the abnormal cells. The cells once detached do not re-adhere because the original place is irreversibly changed into a non-cell-adhesive one, enabling the effective removal of the abnormal cells. In addition, an electrical stimulus and an impact as for a flow cytometer or a sorting device are not present, and the control of the photoreaction wavelength, the light wavelength for microscopic observation, and the exciting light wavelength for fluorescent observation can reduce phototoxicity to cells; thus, the viability of cells can be increased. Further, cells are arranged on a 2-dimensional plane, almost simultaneously exposed to light to each cells, and subjected to microscopic or fluorescent observation; thus, optical axis adjustment for 1-dimensionaly arranging cells and exposing individual cells to a laser as for the flow cytometer is unnecessary.

### Reference Signs List

- 1: Transparent Base Material
- 2: Cell-Adhesive Photocontrollable Material
- 3, 4, 9: Cell
- 5: Second Light Irradiation
- 6: Cut Region between Cells and of Cell-Adhesive Photocontrollable Material
- 7: First Light Irradiation
- 8: Region Changed from Cell-Adhesive One to Non-Cell-Adhesive One by Photoreaction (Non-Cell-Adhesive Region)
- 10, 11: Region which is changed from cell-adhesive to non cell-adhesive by light irradiation
- 69: Cell-Adhesive Region

All publications, patents, and patent applications cited in this specification are intended to be incorporated herein by reference in their entirety.

## Claims

1. A cell-adhesive photocontrollable base material, wherein light irradiation causes the bond dissociation of a photolabile group comprising an O-nitrobenzyl skeleton to irreversibly change the surface of the light-irradiated portion thereof from that of the cell-adhesive material to that of a non-cell-adhesive material.

2. A cell-adhesive photocontrollable base material obtained by film-forming a cell-adhesive photocontrollable material in which a cell-adhesive material is bonded to a non-cell-adhesive material via a photolabile group comprising an O-nitrobenzyl skeleton, on a base material.

3. A cell-adhesive photocontrollable base material, wherein light irradiation causes the bond dissociation of a photolabile group comprising an O-nitrobenzyl skeleton to separate a cell-adhesive material to leave a non-cell-adhesive material.

4. The cell-adhesive photocontrollable base material according to any of claims 1 to 3, wherein the photolabile group comprises a divalent O-nitrobenzyl skeleton represented by general formula (5) below: 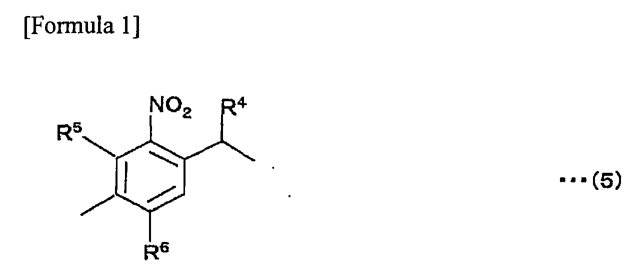 wherein R⁴ represents a group selected from the group consisting of hydrogen, a halogen group, a hydroxy group, an alkyl group, a carboxylic acid group, a carboxylic acid ester group, an amide group, and an amino group; R⁵ and R⁶ each independently represent a group selected from the group consisting of hydrogen, an alkoxy group, and a vinyloxy group; and the mutual positional relationship between meta and para positions may be changed.

5. The cell-adhesive photocontrollable base material according to any one of claims 1 to 3, wherein the cell-adhesive photocontrollable material comprises a structure in which a cell-adhesive group represented by general formula (4) directly or indirectly bonds to a photolabile group represented by general formula (5) at a position on the benzene skeleton or at the benzyl position:
[Formula 2]
-X (4)
wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; and 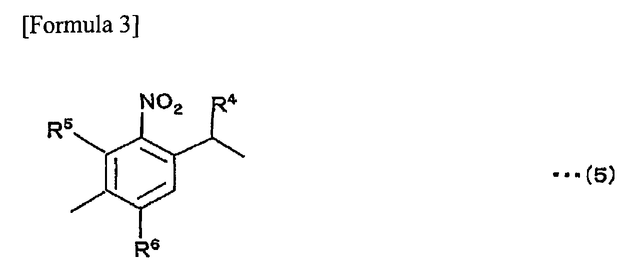 wherein (5) R⁴ represents a group selected from the group consisting of hydrogen, a halogen group, a hydroxy group, an alkyl group, a carboxylic acid group, a carboxylic acid ester group, an amide group, and an amino group; R⁵ and R⁶ each independently represent a group selected from the group consisting of hydrogen, an alkoxy group, and a vinyloxy group; and the mutual positional relationship between meta and para positions may be changed.

6. The cell-adhesive photocontrollable base material according to any one of claims 1 to 3, wherein the cell-adhesive photocontrollable material comprises a structure in which the cell-adhesive group directly or indirectly bonds to the photolabile group on the benzene skeleton via a divalent linking group R⁷, represented by general formula (6) below: 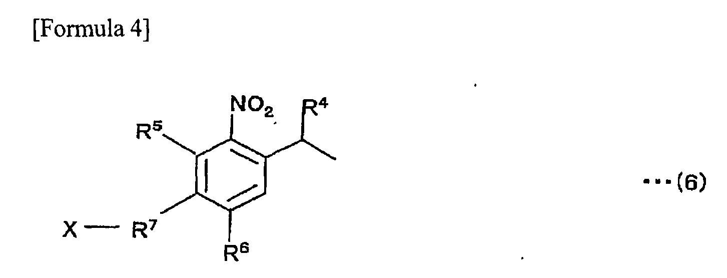 wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; R⁴ represents a group selected from the group consisting of hydrogen, a halogen group, a hydroxy group, an alkyl group, a carboxylic acid group, a carboxylic acid ester group, an amide group, and an amino group; R⁵ and R⁶ each independently represent a group selected from the group consisting of hydrogen, an alkoxy group, and a vinyloxy group; the mutual positional relationship between meta and para positions may be changed; and R⁷ represents a divalent linking group selected from O(CH₂)m, O(CH₂CH₂O)ₘ, OCO(CH₂)ₘ, and OCOCH₂O(CH₂CH₂O)ₘ (where m is an integer of 0 to 20).

7. The cell-adhesive photocontrollable base material according to any one of claims 1 to 3, wherein the cell-adhesive photocontrollable material comprises a structure in which the photolabile group bonds to the cell-adhesive group at the benzyl position, represented by general formula (7) below: 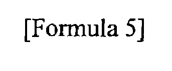 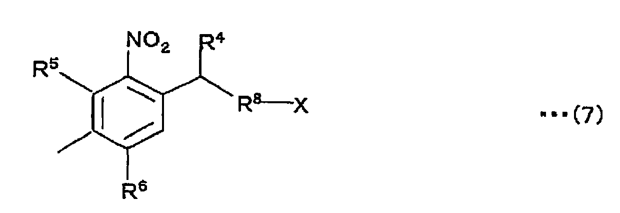 wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group; R⁴ represents a group selected from the group consisting of hydrogen, a halogen group, a hydroxy group, an alkyl group, a carboxylic acid group, a carboxylic acid ester group, an amide group, and an amino group; R⁵ and R⁶ each independently represent a group selected from the group consisting of hydrogen, an alkoxy group, and a vinyloxy group; the mutual positional relationship between meta and para positions may be changed; and R⁸ represents a divalent linking group selected from the group consisting of O, OCO, NH, OCONH, and NHCO.

8. The cell-adhesive photocontrollable base material according to any one of claims 1 to 3, wherein the cell-adhesive photocontrollable material comprises a structure in which a cell-adhesive group represented by general formula (4) directly or indirectly bonds to a photolabile group represented by general formula (5) at the benzene ring or benzyl position of the O-nitrobenzyl skeleton, directly or indirectly bonds to the non-cell-adhesive material:
[Formula 6]
-X (4)
wherein X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group, 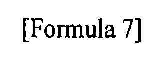 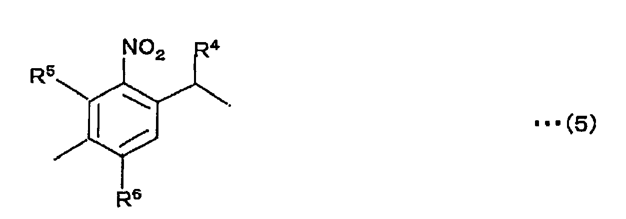 wherein R⁴ represents a group selected from the group consisting of hydrogen, a halogen group, a hydroxy group, an alkyl group, a carboxylic acid group, a carboxylic acid ester group,
an amide group, and an amino group; R⁵ and R⁶ each independently represent a group selected from the group consisting of hydrogen, an alkoxy group, and a vinyloxy group; and the mutual positional relationship between meta and para positions may be changed.

9. The cell-adhesive photocontrollable base material according to any one of claims 1 to 3, wherein the cell-adhesive photocontrollable material comprises a structure represented by general formula (8) below: 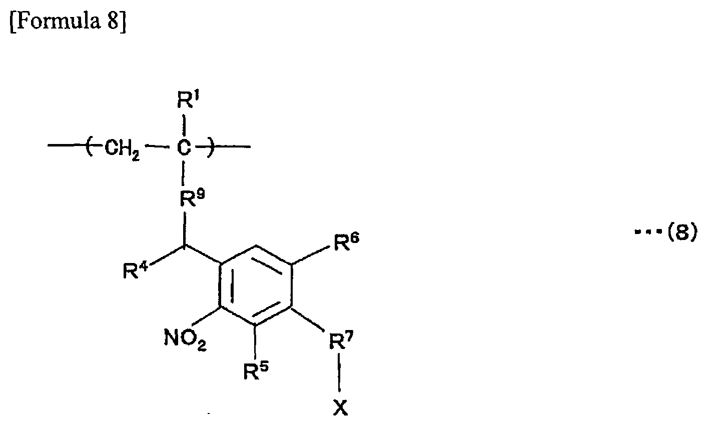 wherein R¹ represents hydrogen or a methyl group; R⁴ represents a group selected from the group consisting of hydrogen, a halogen group, a hydroxy group, an alkyl group, a carboxylic acid group, a carboxylic acid ester group, an amide group, and an amino group; R⁵ and R⁶ each independently represent a group selected from the group consisting of hydrogen, an alkoxy group, and a vinyloxy group; the mutual positional relationship between meta and para positions may be changed; R⁷ represents a divalent linking group selected from the group consisting of O(CH₂)ₘ, O(CH₂CH₂O)ₘ, OCO(CH₂)ₘ, and OCOCH₂O(CH₂CH₂O)ₘ (where m is an integer of 0 to 20); R⁹ represents a divalent linking group selected from the group consisting of O, CO₂, CONH, CO₂(CH₂CH₂O)ₚ, CO₂(CH₂CH₂)ₚO, CONH(CH₂CH₂O)ₚ, and CONH(CH₂CH₂)ₚO (p is an integer of 1 to 20); and X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group.

10. The cell-adhesive photocontrollable base material according to any of claims 1 to 3, wherein the cell-adhesive photocontrollable material comprises a structure represented by general formula (9) below: 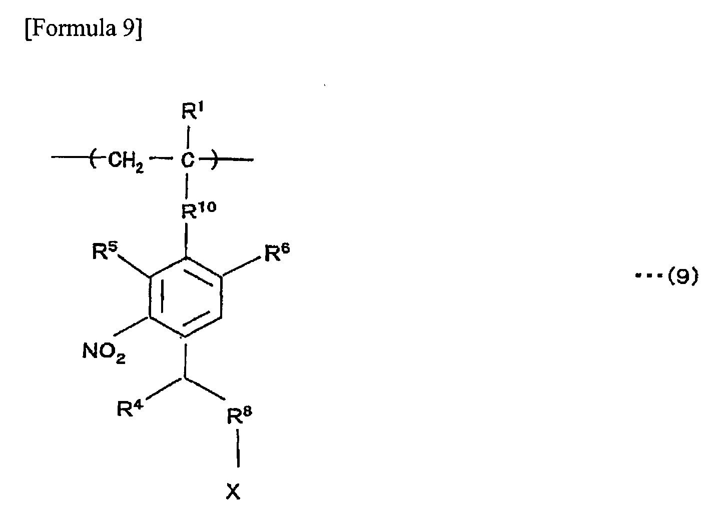 wherein R¹ represents hydrogen or a methyl group; R⁴ represents a group selected from the group consisting of hydrogen, a halogen group, a hydroxy group, an alkyl group, a carboxylic acid group, a carboxylic acid ester group, an amide group, and an amino group; R⁵ and R⁶ represent groups selected from the group consisting of hydrogen, an alkoxy group, and a vinyloxy group; the mutual positional relationship between meta and para positions may be changed; R⁸ represents a divalent linking group selected from the group consisting of O, OCO, NH, OCONH, and NHCO; R¹⁰ represents a divalent linking group selected from the group consisting of O, CO₂, CONH, CO₂(CH₂CH₂O)ₚ, CO₂(CH₂CH₂)ₚO, CONH(CH₂CH₂O)ₚ, and CONH(CH₂CH₂)ₚO (p is an integer of 1 to 20); and X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group.

11. The cell-adhesive photocontrollable base material according to any one of claims 1 to 3, wherein the cell-adhesive photocontrollable material comprises any of (meth)acrylic ester copolymers represented by general formulas (1) and (8) below; general formulas (1) and (9) below; general formulas (1), (8), and (2) below; or general formulas (1), (9), and (2) below: 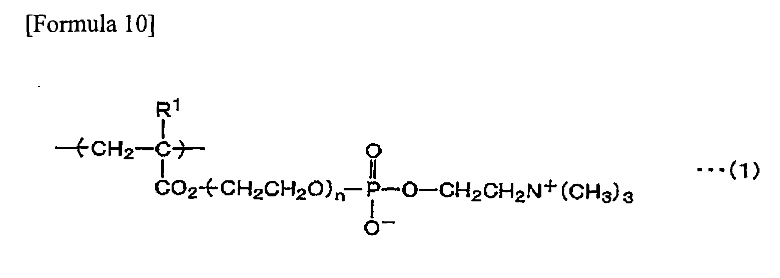 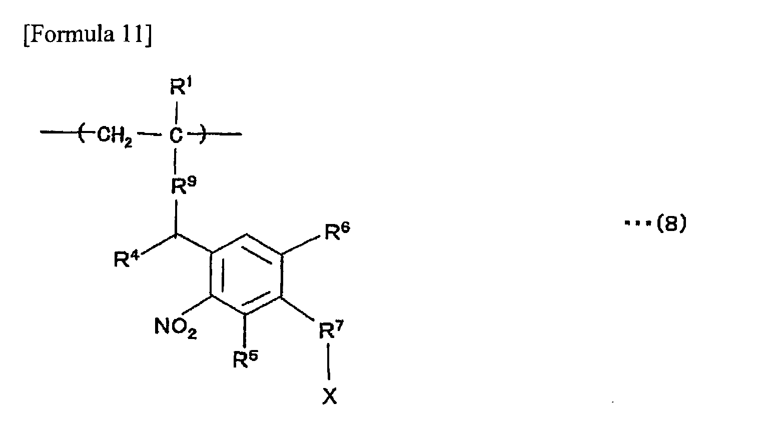 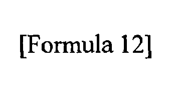 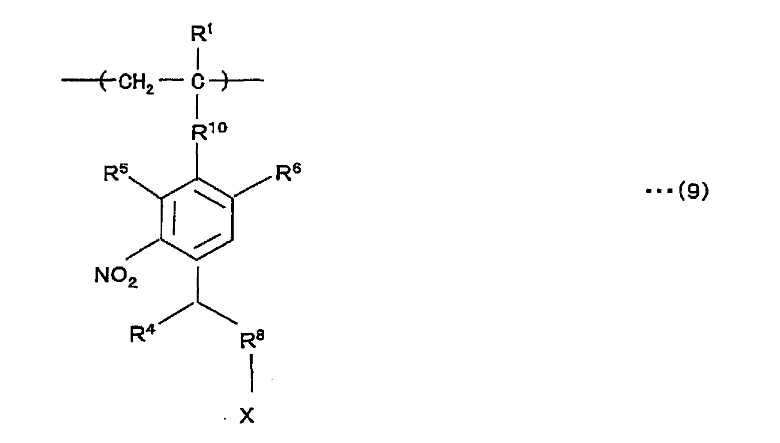 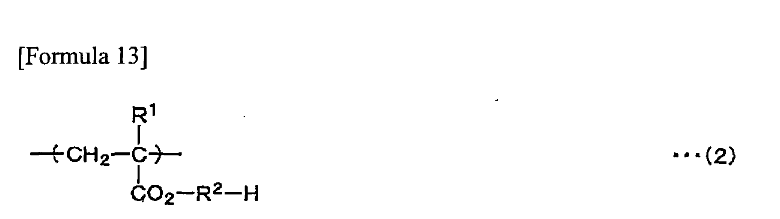 wherein R¹ represents hydrogen or a methyl group; R² represents 1 to 20 alkylene groups or to 20 polyoxyethylene groups; R⁴ represents a group selected from the group consisting of hydrogen, a halogen group, a hydroxy group, an alkyl group, a carboxylic acid group, a carboxylic acid ester group, an amide group, and an amino group; R⁵ and R⁶ each independently represent a group selected from the group consisting of hydrogen, an alkoxy group, and a vinyloxy group; the mutual positional relationship between meta and para positions may be changed; R⁷ represents a divalent linking group selected from the group consisting of O(CH₂)m, O(CH₂CH₂O)ₘ, OCO(CH₂)ₘ, and OCOCH₂O(CH₂CH₂O)ₘ (where m is an integer of 0 to 20); R⁸ represents a divalent linking group selected from the group consisting of O, OCO, NH, OCONH, and NHCO; R⁹ and R¹⁰ represent divalent linking groups selected from the group consisting of O, CO₂, CONH, CO₂(CH₂CH₂O)ₚ, CO₂(CH₂CH₂)ₚO, CONH(CH₂CH₂O)ₚ, and CONH(CH₂CH₂)ₚO (p is an integer of 1 to 20); and X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group.

12. The cell-adhesive photocontrollable base material according to any one of claims 1 to 3, wherein the cell-adhesive photocontrollable material has a (meth)acrylic acid ester comprising an alkoxysilane copolymerized at the side chain.

13. The cell-adhesive photocontrollable base material according to any one of claims 1 to 3, wherein the cell-adhesive photocontrollable material is formed by film-forming an alkoxysilane represented by general formula (10) below on a base material and then introducing a cell-adhesive group X: 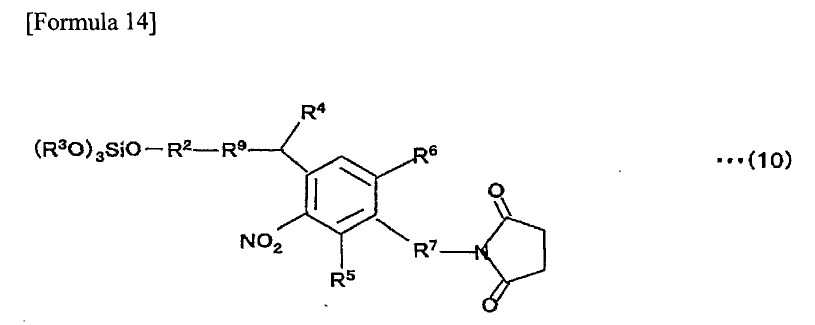 wherein R² represents Cl to C20 alkylene groups or 1 to 20 polyoxyethylene groups; R³ represents hydrogen or an alkyl group; R⁴ represents a group selected from the group consisting of hydrogen, a halogen group, a hydroxy group, an alkyl group, a carboxylic acid group, a carboxylic acid ester group, an amide group, and an amino group; R⁵ and R⁶ each independently represent a group selected from the group consisting of hydrogen, an alkoxy group, and a vinyloxy group; the mutual positional relationship between meta and para positions may be changed; R⁷ represents a divalent linking group selected from the group consisting of O(CH₂)ₘ, O(CH₂CH₂O)ₘ, OCO(CH₂)ₘ, and OCOCH₂O(CH₂CH₂O)ₘ (where m is an integer of 0 to 20); R⁹ represents a divalent linking group selected from the group consisting of O, CO₂, CONH, CO₂(CH₂CH₂O)ₚ, CO₂(CH₂CH₂)ₚO, CONH(CH₂CH₂O)ₚ, and CONH(CH₂CH₂)ₚO (p is an integer of 1 to 20); and X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group.

14. The cell-adhesive photocontrollable base material according to any one of claims 1 to 3, wherein the cell-adhesive photocontrollable material is formed by film-forming an alkoxysilane represented by general formula (11) below on a base material and then introducing a cell-adhesive group X: 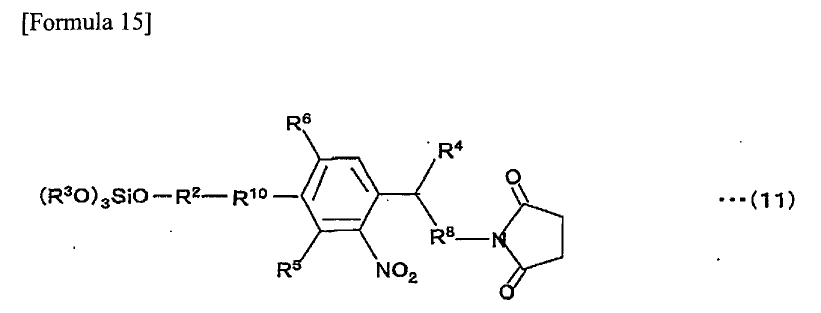 wherein R² represents C1 to C20 alkylene groups or 1 to 20 polyoxyethylene groups; R³ represents hydrogen or an alkyl group; R⁴ represents a group selected from the group consisting of hydrogen, a halogen group, a hydroxy group, an alkyl group, a carboxylic acid group, a carboxylic acid ester group, an amide group, and an amino group; R⁵ and R⁶ represent groups selected from the group consisting of hydrogen, an alkoxy group, and a vinyloxy group; the mutual positional relationship between meta and para positions may be changed; R⁸ represents a divalent linking group selected from the group consisting of O, OCO, NH, OCONH, and NHCO; R¹⁰ represents a divalent linking group selected from the group consisting of O, CO₂, CONH, CO₂(CH₂CH₂O)ₚ, CO₂(CH₂CH₂)ₚO, CONH(CH₂CH₂O)ₚ, and CONH(CH₂CH₂)ₚO (p is an integer of 1 to 20); and X represents a group selected from the group consisting of a carboxylic acid, an alkyl mono- or polycarboxylate group, an amino group, a mono- or polyaminoalkyl group, an amide group, an alkyl mono- or polyamide group, a hydrazide group, an alkyl mono- or polyhydrazide group, an amino acid group, a polypeptide group, and a nucleic acid group.
